# EUROPEAN PATENT APPLICATION

(11) **EP 2 711 005 A1**
(43) Date of publication of application: **26.03.2014**
(21) Application number: 12768645.9
(22) Date of filing: 31.03.2012
(51) Int. Cl.: A61K 31/357, A61K 33/26, A61K 47/02, A61K 47/06, A61K 9/52, A61P 35/00

(54) **COMPOUND DUAL-RELEASE CAPSULE FORMULATION COMPRISED OF BROMODIHYDROARTEISININ AND FE2+ AGENT**

(30) Priority: 08.04.2011 CN 201110089000
(71) Applicant: Shi, Yanyu, Chengdu, Sichuan 610066 (CN)
(72) Inventor: Shi, Yanyu, Chengdu, Sichuan 610066 (CN)
(74) Representative: Zaboliene, Reda
(86) International application number: PCT/CN2012/073436
(87) International publication number: WO 2012/136125

(57) **Abstract**

Disclosed is a compound dual-release capsule formulation consisting of bromo-dihydroartemisinin and a Fe²⁺ agent and comprising micropills dissolving in the intestine and micropills for rapid dissolution in the stomach, said micropills dissolving in the intestine consisting of cores of bromo-dihydroartemisinin and pharmaceutically acceptable excipients enrobed in intestinal-release coating, and said rapid-release micropills dissolving in the stomach consisting of Fe²⁺ agent and pharmaceutically acceptable excipients. The micropills of intestinal-release bromo-dihydroartemisinin and the micropills of rapid-release Fe²⁺ agent are packaged together in gelatin capsules dissolving in the stomach, thus constituting a dual-release formulation.

## Description

### FIELD OF THE INVENTION

The invention belongs to the field of pharmacy and relates to a compound dual-release formulation prepared by encapsulating bromo-dihydroartemisinin (Br-DHA) enteric-coated pellets and Fe²⁺ agent gastric immediate-release pellets into a capsule. The formulation of the invention is mainly used for the treatment of broad-spectrum cancers.

### DESCRIPTION OF THE RELATED ART

The inventor's early research, see Chinese Patent Application Number CN200610144186.3 (Authorized Patent Publication Number CN100480250C) which is incorporated herein in its entirety, discloses that bromo-dihydroartemisinin (i.e., brominated dihydroartemisinin or Br-DHA) has more efficient anti-cancer effects than the parent nucleus thereof - Artemisinin. The anti-cancer mechanism of bromo-dihydroartemisinin is that Fe²⁺ contained in cancer cells is much higher than that in normal cells because cancer cell division needs a lot of Fe²⁺ to replicate DNA. The artemisinin moiety in bromo-dihydroartemisinin molecule has a unique endoperoxide-bridge structure and the endoperoxide-bridge will be split once contacting with Fe²⁺ to generate electrically charged atoms as called "free radicals". The charged radicals will attack cell membrane to result in death or apoptosis of cancer cells. Thus, Fe²⁺ and the endoperoxide-bridge radical are indivisible for activating anti-cancer function. In clinical practice, for convenient administration and better anti-cancer effects for patients, it is preferred to formulate a Fe²⁺ agent together with bromo-dihydroartemisinin as a compound preparation. However, it is a problem that the parietal cells will be damaged if the Fe²⁺ agent directly contacts with (reacts with) bromo-dihydroartemisinin in stomach.

### BRIEF SUMMARY OF THE INVENTION

In view of the above problems, the present invention is to provide a compound dual-release capsule formulation consisting of bromo-dihydroartemisinin and Fe²⁺ agent. According to the characteristics of the Fe²⁺ agent being absorbed in stomach under gastric acid and the bromo-dihydroartemisinin beginning to be absorbed in jejunum, it is envisaged that the bromo-dihydroartemisinin is pelletized and coated by enteric-coating, and the Fe²⁺ agent is formed as conventional gastric-soluable pellets. In this way, the bromo-dihydroartemisinin is sustained to release in intestine and the Fe²⁺ agent is immediate-released in stomach, so that the side effect of hurting stomach can be avoided because the both components are completely separated from each other in time and in absorption sites.

To achieve the above object, the present invention employs the following technical solution. A compound dual-release capsule formulation consisting of bromo-dihydroartemisinin and Fe²⁺ agent, comprises a enteric-coated pellet and a gastric immediate-release pellet, wherein the enteric-coated pellet is pelletized by forming a core from bromo-dihydroartemisinin and pharmaceutically acceptable excipients and then coating the core with a enteric-coating material; and the gastric immediate-release pellet is made from a Fe²⁺ agent and pharmaceutically acceptable excipients, wherein the bromo-dihydroartemisinin enteric-coated pellet and the Fe²⁺ agent gastic immediate-release pellet are encapsulated into an ordinary gastic-soluable capsule shell to form the compound dual-release capsule formulation, in which the formulation comprising 15-20% of bromo-dihydroartemisinin and 1.5-2.0% of Fe²⁺ by mass, and the balance of the excipients.

In the present invention, the Fe²⁺ agent can be ferrous sulfate, ferrous lactate or ferrous succinate, and preferably is ferrous sulfate heptahydrate (FeSO4•7H2O). The compound dual-release formulation consisting of bromo-dihydroartemisinin and Fe²⁺ agent comprises 15-20% of bromo-dihydroartemisinin and 7.5-10% of ferrous sulfate heptahydrate by mass, and the balance of excipients.

The present invention provides a formula of a core and a coating of the bromo-dihydroartemisinin enteric-coated pellet in that the core of the bromo-dihydroartemisinin pellet is made from bromo-dihydroartemisinin with lactose, starch and magnesium stearate by the conventional pellet-core manufacture process, and the enteric-coating material of the bromo-dihydroartemisinin enteric-coated pellet is made from enteric acrylic acid, polyethylene terephthalate, Tween-80, castor oil and 80% ethanol by the conventional pellet-coating process.

The enteric-coating material of the bromo-dihydroartemisinin enteric-coated pellet is also suitable for using commercially available enteric-coating materials as the sustained-release coating.

The present invention also provides a formula of a core of the Fe²⁺ agent gastic immediate-release pellet in that the core of the Fe²⁺ agent gastic immediate-release pellet is made from the Fe²⁺ agent with lactose, dextrin and 10% PVP in ethanol by the conventional pellet-core manufacture process. The resulting pellet is gastic immediate-releasing. In the present invention, the Fe²⁺ agent gastic immediate-release pellet can be coated with or without gastic-coating if necessary.

The both pellets (i.e., the bromo-dihydroartemisinin enteric-coated pellet and the Fe²⁺ gastic immediate-release pellet) can be coated with different colored outer-coating respectively to show their difference or without the colored outer-coating, but their bulk density must be consistent, and then the both can be dry-mixed according to each clinical demanded content ratio and filled into the capsule. Such dosage form is applicable for treatment of a broad spectrum of cancer.

The inventor selected the combination of ferrous sulfate and bromo-dihydroartemisinin (compound formulation) to perform the following in vitro anti-tumor test.

Methods: liver cancer cells HCC MHCC97 in logarithmic phase are prepared into 8×10³ suspension after normal digestion and then seeded into 96-well cell culture plate. After 24h cell culture, the culture medium is sucked out and mixed with ferrous sulfate and bromo-dihydroartemisinin (Br-DHA) in a certain proportion, and the mixture is re-added into cell culture plate and cultured in an incubator for 3 days. Blank control wells and Br-DHA control wells are also prepared and cultured in the incubator for 3 days. The cell counting results by the conventional method are shown in Table 1.

**[Table 1]**

| Groups | Number of specimens | Dosage of drugs | | Cell Survival Rate (CSR) % |
|---|---|---|---|---|
| | | Ferrous sulfate (nM) | Br-DHA (nM) | |
| Blank control | 4 | - | - | 100.0±8.9 |
| Br-DHA only | 4 | - | 400 | 80.2±16.5 |
| Compound formulation | 4 | 100 | 400 | 16.3±3.8 |
| | 4 | 100 | 200 | 23.7±8.9 |
| | 4 | 100 | 100 | 26.6±12.1 |
| | 4 | 100 | 50 | 33.1±4.5 |

As can be seen, the experiment shows that the compound formulation of Fe²⁺ agent (ferrous sulfate) and Br-DHA has a significant anti-tumor effect in vitro.

The present invention has the advantages that: the bromo-dihydroartemisinin enteric-coated pellets and the Fe²⁺ gastic immediate-release pellets can be administered at the same time in the same capsule by patients and easy to take; the bromo-dihydroartemisinin enteric-coated pellets and the Fe²⁺ gastic immediate-release pellets are encapsulated into capsules so that the Fe²⁺ agent gastic immediate-release pellets are absorbed in stomach under gastric acid and the bromo-dihydroartemisinin enteric-coated pellets begin to be absorbed in jejunum so as to avoid the reaction of Br-DHA with Fe²⁺ in stomach and the damage effects on parietal cells. Meanwhile, bromo-dihydroartemisinin is sustained-releasing and Fe²⁺ agent is immediately-releasing, so that the side effect of hurting stomach can be avoided because the both components are completely separated from each other in time and in absorption sites.

### DESCRIPTION OF THE PREFERRED EMBODIMENTS

The present invention will be described by the following examples. The pelleting and coating processes are well known techniques in pharmacy industry. The following examples mainly describe the embodiments of the compound dual-release formulation, but the present invention is not limited to the following examples.

### Example 1

### Preparation of bromo-dihydroartemisinin enteric-coated pellets:

Bromo-dihydroartemisinin 33g together with lactose 50g, starch 28g and magnesium stearate 3g were weighted and pelletized into pellet cores by conventional pelleting process. The coating material thereof used enteric acrylic acid 62g, polyethylene terephthalate 1 g, Tween-80 0.8g, castor oil 10ml, 80% ethanol 20ml. The enteric acrylic acid was dissolved in 80% ethanol, and then the remaining coating material components were added to obtain the coating material after thorough mixing. The pellet cores were coated with such coating material to prepare the bromo-dihydroartemisinin enteric-coated pellets with pellet size of 30 mesh. The coating material of such formula can has a release time sustained for 2-3 hours and the dissolution pH of 5.3-6.5, without any dependency on the coating thickness, which complies with the clinical demand of dissolubility in intestinal tract and indissolubility in gastic acid.

### Preparation of Fe²⁺ agent (ferrous succinate) gastic immediately-release pellets:

The ferrous succinate gastic immediately-release pellets with pellet size of 30 mesh were prepared by weighting ferrous succinate 20g, lactose 25g and dextrin 25g as a solid dispersion carrier and an appropriate amount of 10% PVP in ethanol as a binder through solvent pelleting process.

The bromo-dihydroartemisinin enteric-coated pellets were coated with colored outer coating and the ferrous succinate gastic immediately-release pellets were coated without outer coating, followed by dry blending (fully mixed) and filling 1,000 capsules with them.

The gastic juice and the intestinal juice are formulated according to the guideline and procedure specified in the appendix of Chinese Pharmacopoeia (2005 edition, volume II) and tested by dissolution rate detection method. The results are shown in Table 2.

**[Table 2]**

| Test items | Dissolution rate in gastic juice | Dissolution rate in intestinal juice |
|---|---|---|
| Ferrous succinate gastic immediately-release pellets | 1hr, 60% | 2hrs, 87% |
| Bromo-dihydroartemisinin enteric-coated pellets | 8hrs, indissoluble | 2hrs, 62% |

### Example 2

### Preparation of bromo-dihydroartemisinin enteric-coated pellets:

Bromo-dihydroartemisinin 40g together with lactose 72g and magnesium stearate 5g were weighted and pelletized into pellet cores. The coating material thereof used enteric acrylic acid 6g, polyethylene terephthalate 0.8g, Tween-80 1 g, castor oil 10ml, 80% ethanol 20ml. The enteric acrylic acid was dissolved in 80% ethanol, and then the remaining coating material components were added and fully mixed, followed by coating with the resulting coating material to obtain the bromo-dihydroartemisinin enteric-coated pellets with pellet size of 30 mesh.

### Preparation of Fe²⁺ agent (ferrous sulfate) gastic immediately-release pellets:

Ferrous sulfate 20g, lactose 30g and dextrin 10g were weighted as a solid dispersion carrier and an appropriate amount of 10% PVP in ethanol was weighted as a binder to form pellets with pellet size of 30 mesh by solvent pelleting process.

The bromo-dihydroartemisinin enteric-coated pellets were coated with colored outer coating and the ferrous sulfate gastic immediately-release pellets were coated with white outer coating, followed by dry blending (fully mixed) and filling 1,000 capsules with them.

The gastic juice and the intestinal juice are formulated according to the guideline and procedure specified in the appendix of Chinese Pharmacopoeia (2005 edition, volume II) and tested by dissolution rate detection method. The results are shown in Table 3.

**[Table 3]**

| Test items | Dissolution rate in gastric juice | Dissolution rate in intestinal juice |
|---|---|---|
| Ferrous sulfate gastic immediately-release pellets | 1hr, 95% | 2hrs, 90% |
| Bromo-dihydroartemisinin enteric-coated pellets | 8hrs, indissoluble | 2hrs, 90% |

### Example 3

### Preparation of bromo-dihydroartemisinin enteric-coated pellets:

Bromo-dihydroartemisinin 44g together with lactose 50g, starch 23g and magnesium stearate 3g were weighted and pelletized into pellet cores. The coating material thereof used enteric acrylic acid 6g, polyethylene terephthalate 1.2g, Tween-80 0.8g, castor oil 5ml, 80% ethanol 25ml. The enteric acrylic acid was dissolved in 80% ethanol, and then the remaining coating material components were added and fully mixed, followed by coating with the resulting coating material to obtain the bromo-dihydroartemisinin enteric-coated pellets with pellet size of 30 mesh.

### Preparation of Fe²⁺ agent (ferrous lactate) gastic immediately-release pellets:

Ferrous lactate 22g, lactose 20g and dextrin 20g were weighted as a solid dispersion carrier and an appropriate amount of 10% PVP in ethanol was weighted as a binder to form the ferrous lactate gastic immediately-release pellets with pellet size of 30 mesh by solvent pelleting process.

Both of the bromo-dihydroartemisinin enteric-coated pellets and the ferrous lactate gastic immediately-release pellets were dry blended (fully mixed) and 1,000 capsules were filled with the resulting mixture.

The gastic juice and the intestinal juice are formulated according to the guideline and procedure specified in the appendix of Chinese Pharmacopoeia (2005 edition, volume II) and tested by dissolution rate detection method. The results are shown in Table 4.

**[Table 4]**

| Test items | Dissolution rate in gastic juice | Dissolution rate in intestinal juice |
|---|---|---|
| Ferrous lactate gastic immediately-release pellets | 1hr, 80% | 2hrs, 85% |
| Bromo-dihydroartemisinin enteric-coated pellets | 8hrs, indissoluble | 2hrs, 86% |

### Performing the following in vivo anti-tumor experiments by using the compound dual release capsule formulation of the present invention:

Methods: 40 rats were prepared with each half of male and female. Human lung cancer cell line A549 was inoculated subcutaneously in the shoulders of the rats. 2 weeks later, when the tumor has a volume of about 1cm³, the rats were randomly equally divided into 4 groups, i.e., model group, Taxol (Paclitaxel) control group, dihydroartemisinin group, compound dual-release capsule formulation prepared in Example 2 group.

In the Taxol control group, the rats were injected with Taxol of calculated dose according to clinical dose. In dihydroartemisinin group, the rats were intragastrically administered with carboxymethylcellulose suspension comprising dihydroartemisinin in dose of 0.898mg/day per rat. In the compound dual-release capsule formulation prepared in Example 2 group, the compound dual-release capsules prepared in Example 2 were broken up and removed the bromo-dihydroartemisinin enteric-coated pellets and the ferrous sulfate immediate-release pellets contained in the capsules, and then the rats were intragastrically administered with carboxymethylcellulose suspension comprising 4.4mg of the bromo-dihydroartemisinin enteric-coated pellets and 1.74mg of the ferrous sulfate immediate-release pellets, each in morning and afternoon. In the model geoup, the rats were intragastrically administered with carboxymethylcellulose suspension only. All the rats were measured of the tumor volume two times in a week and killed in four weeks for weighting the tumors. The results are shown in Table 5.

**[Table 5]**

| Groups | Tumor volume (mm³) | Tumor weight (g) |
|---|---|---|
| Model group | 2059.52±23.35 | 24.40±17.13 |
| Taxol group | 1769.36±10.11 | 18.35±9.99 |
| Dihydroartemisinin group | 2285.62±17.66 | 23.90±5.51 |
| Compound dual-release capsule (Example 2) group | 1008.53±12.21 | 13.50±8.78 |

The experiment shows that the dual-release formulation of Fe2+ agent and Br-DHA has significant in vivo anti-tumor activity which is much better than Taxol.

The tumor bodies separated from the rats administered with the compound dual-release capsule formulation in Example 2 were biopsied and observed. It were found that the section of the tumor body shows off-white color, flocculent-like in center, large area of non-cell structure zone, internal necrotic area of about 76.47%, and local myxedema surrounding the necrotic area. The biopsy further shows that the formulation in Example 2 has a significant function of killing cancer cells.

Further modifications in addition to those described above may be made to the embodiments described herein without departing from the spirit and scope of the invention. Accordingly, although specific embodiments have been described, these are examples only and are not limiting upon the scope of the invention.

## Claims

1. A compound dual-release capsule formulation consisting of bromo-dihydroartemisinin and Fe²⁺ agent, is **characterized in that** it comprises a enteric-coated pellet and a gastric immediate-release pellet, wherein the enteric-coated pellet is pelletized by forming a core from bromo-dihydroartemisinin and pharmaceutically acceptable excipients, and then coating the core with a enteric-coating material; and the gastric immediate-release pellet is made from Fe²⁺ agent and pharmaceutically acceptable excipients, wherein the bromo-dihydroartemisinin enteric-coated pellet and the Fe²⁺ agent gastic immediate-release pellet are encapsulated into a ordinary gastic-soluable capsule shell to form the compound dual-release capsule formulation, in which the formulation comprising 15-20% of bromo-dihydroartemisinin and 1.5-2.0% of Fe²⁺ by mass, and the balance of the excipients.

2. The compound dual-release capsule formulation consisting of bromo-dihydroartemisinin and Fe²⁺ agent in accordance with Claim 1, wherein the Fe²⁺ agent is ferrous sulfate, ferrous lactate or ferrous succinate.

3. The compound dual-release capsule formulation consisting of bromo-dihydroartemisinin and Fe²⁺ agent in accordance with Claim 2, wherein the compound dual-release capsule formulation comprises 15-20% of bromo-dihydroartemisinin and 7.5-10% of ferrous sulfate heptahydrate by mass.

4. The compound dual-release capsule formulation consisting of bromo-dihydroartemisinin and Fe²⁺ agent in accordance with anyone of Claims 1 to 3, wherein the core of the bromo-dihydroartemisinin enteric-coated pellet is made from bromo-dihydroartemisinin with lactose, starch and magnesium stearate.

5. The compound dual-release capsule formulation consisting of bromo-dihydroartemisinin and Fe²⁺ agent in accordance with anyone of Claims 1 to 3, wherein the enteric-coating material of the bromo-dihydroartemisinin enteric-coated pellet is made from enteric acrylic acid, polyethylene terephthalate, Tween-80, castor oil and 80% ethanol.

6. The compound dual-release capsule formulation consisting of bromo-dihydroartemisinin and Fe²⁺ agent in accordance with anyone of Claims 1 to 3, wherein the enteric-coating material of the bromo-dihydroartemisinin enteric-coated pellet is also suitable for using commercially available enteric-coating materials as the sustained-release coating.

7. The compound dual-release capsule formulation consisting of bromo-dihydroartemisinin and Fe²⁺ agent in accordance with anyone of Claims 1 to 3, wherein the core of the Fe²⁺ agent gastic immediate-release pellet is made from the Fe²⁺ agent with lactose, dextrin and 10% PVP in ethanol.

8. A use of a compound dual-release capsule formulation consisting of bromo-dihydroartemisinin and Fe²⁺ agent as an anti-tumor drug.
